# FASCICULE DE BREVET EUROPEEN

(11) **EP 0 980 263 B1**
(45) Date de publication et mention de la délivrance du brevet: **25.09.2002**
(21) Numéro de dépôt: 98928391.6
(22) Date de dépôt: 29.05.1998
(51) Int. Cl.: A61K 48/00, A61K 38/46

(54) **PRODUIT DE COMBINAISON ASSOCIANT UN ACIDE NUCLEIQUE A UNE SUBSTANCE DESORGANISANT LA MATRICE EXTRACELLULAIRE POUR LA THERAPIE GENIQUE**
KOMBINATIONSPRODUKT, ENTHALTEND EINE NUKLEINSÄURE UND EINE SUBSTANZ, DIE DIE EXTRAZELLULÄREMATRIX DESORGANISIERT, ZUR VERWENDUNG IN GENTHERAPIE
COMBINED PRODUCT ASSOCIATING A NUCLEIC ACID WITH A SUBSTANCE BREAKING UP THE EXTRACELLULAR MATRIX FOR GENE THERAPY

(30) Priorité: 29.05.1997 FR 9706600
(43) Date de publication de la demande: 23.02.2000
(73) Titulaire: TRANSGENE S.A., 67000 Strasbourg (FR); ASSOCIATION FRANCAISE CONTRE LES MYOPATHIES, F-75013 Paris (FR)
(72) Inventeur: BRAUN, Serge, F-67120 Dorlisheim (FR)
(74) Mandataire: Warcoin, Jacques
(86) Numéro de dépôt international: FR9801084
(87) Numéro de publication internationale: WO98053853

(56) Documents cités:
- WO-A-91/12329
- WO-A-95/26718
- DUBENSKY ET AL: "DIRECT TRANSFER OF VIRAL AND PLASMID DNA INTO THE LIVER OR SPLEEN OF MICE" PROCEEDINGS OF THE NATIONAL ACADEMY OF SCIENCES,USA, vol. 81, 1984, pages 7529-7533, XP002057236 cité dans la demande

## Description

La présente invention concerne un produit associant un acide nucléique et une substance entraînant une désorganisation de la matrice extracellulaire d'une cellule, notamment la hyaluronidase, pour une utilisation simultanée, consécutive ou étalée dans le temps, ledit acide nucléique étant sous la forme d'une particule virale infectieuse ou d'un vecteur synthétique, sélectionné parmi les vecteurs à lipides cationiques ou à polymères cationiques, étant entendu que ladite substance n'est pas la collagénase. Elle a également pour objet l'utilisation d'un tel produit dans le but de favoriser le transfert de l'acide nucléique en question dans une cellule ou un organisme hôte. Elle est particulièrement utile dans le domaine du transfert de gène ou de la thérapie génique.

La matrice extracellulaire est constituée de molécules protéiques et polysaccharidiques assemblées en un réseau dense organisé dans l'espace extracellulaire de la plupart des tissus. Elle joue un rôle physiologique important de maintien de l'architecture tissulaire, de réservoir de facteurs trophiques, de chemoattracteurs et de facteurs d'attachement de cellules. Le hyaluronan (ou acide hyaluronique) est un constituant ubiquitaire de la matrice extracellulaire des vertébrés. Ce polysaccharide linéaire à base d'acide glucuronique et de glucosamine (D-glucuronic acid 1-β-3)N-acetyl-D-glucosamine(1-b-4)] peut influencer les caractéristiques physicochimiques des matrices de par sa propriété de former des solutions très visqueuses. L'acide hyaluronique interagit également avec divers récepteurs et protéines de liaison situés à la surface des cellules. Il est impliqué dans de nombreux processus biologiques tels que la fertilisation, le développement embryonnaire, la migration cellulaire et la différenciation, la cicatrisation, l'inflammation, la croissance tumorale et la formation de métastases.

L'acide hyaluronique est hydrolysé par la hyaluronidase. Son hydrolyse entraîne une désorganisation de la matrice extracellulaire. La hyaluronidase est présente dans de nombreux liquides biologiques de l'organisme. Produite par l'acrosome des spermatozoïdes, elle assure leur pénétration dans l'ovule lors de la fécondation. Durant l'embryogenèse, elle favorise la migration des cellules embryonnaires vers les territoires qu'elles doivent coloniser.

Outre son action dans la plasticité et la différenciation tissulaire, le couple acide hyaluronique/hyaluronidase serait également impliqué dans certains processus pathologiques. Ainsi, cette enzyme est mise à profit par les cellules cancéreuses pour l'extension des tumeurs et l'angiogénèse qui assure le support nutritif de ces tumeurs. Co-sécrétée dans de nombreux venins (serpents, lézards, poissons, scorpions, abeilles, araignées...), elle augmenterait leur diffusion dans l'organisme de la proie. Elle intervient également dans l'action fusogène de virus comme le MLV (Moloney leukemia virus) ou le CAEV (Caprine arthritis encephalitis virus). Elle pourrait également agir en tant qu'agent de diffusion de virus lors d'une contamination bactérienne comme dans le cas des infections à Herpes (Romano et Moisseiev, Metab. Pediatr. Syst. Ophtalmol. 1982, *6*: 361-365).

Par ailleurs, la hyaluronidase est utilisée depuis de nombreuses années en clinique humaine pour diverses applications : par exemple comme antioedémateux (Lasonil, Thiomucase), agent de diffusion de médicaments injectés par voie intramusculaire ou sous-cutanée (Hyaluronidase Choay), anti-cancéreux, dans la formulation d'anesthésiques locaux (Lewis-Smith, Br. J. Plast. Surg. 1986, *39*: 554-558) ou encore en tant qu'agent réducteur de l'atteinte myocardique à la suite d'infarctus. La possibilité d'utiliser la hyaluronidase dans le domaine de la transfection d'ADN a déjà été évoquée par Dubensky et al. (Proc. Natl. Acad. Sci. USA, 1984, *81*: 7529-7533). Ce document démontre une transfection plus uniforme *in vivo* par co-injection d'ADN plasmidique et d'un mélange de collagénase et de hyaluronidase. Cependant, l'effet bénéfique n'est pas obtenu avec un ADN plasmidique n'ayant pas été au préalable précipité au phosphate de calcium. De même, WO 95/26718 concerne une méthode pour transférer de l'ADN exclusivement nu dans des cellules qui consiste à utiliser un agent qui facilite la pénétration dudit ADN nu à l'intérieur des cellules. Ainsi l'exploitation de cette technologie en thérapie génique utilisant des vecteurs non précipités ou des particules virales est difficilement envisageable.

La présente invention vise à étendre ie potentiel thérapeutique de la hyaluronidase au domaine du transfert de gènes, notamment en thérapie génique. Il est important de pouvoir disposer d'outils favorisant la distribution des vecteurs de gènes ou l'expression de ces derniers au sein de l'organisme hôte pour améliorer l'efficacité de cette nouvelle technologie. La présente invention apporte une solution avantageuse à ce problème. On a maintenant mis en évidence les capacités de la hyaluronidase à favoriser le transfert ou l'expression d'un acide nucléique dans une cellule ou un organisme hôte. Comme le montrent les exemples qui suivent, l'administration intramusculaire d'une solution de hyaluronidase quelques heures avant l'injection dans le même muscle d'adénovirus recombinant augmente sensiblement l'expression du gène recombinant. Cette utilisation combinée améliore l'effet thérapeutique et permet l'emploi de doses réduites de vecteurs.

C'est pourquoi la présente invention a pour objet un produit de combinaison comprenant au moins une substance entraînant une désorganisation d'une matrice extracellulaire d'un hôte et au moins un acide nucléique d'intérêt, pour une administration simultanée, consécutive ou étalée dans le temps, ledit acide nucléique étant sous la forme d'une particule virale infectieuse ou d'un vecteur synthétique, sélectionné parmi les vecteurs à lipides cationiques ou à polymères cationiques, étant entendu que ladite substance n'est pas la collagénase.

Le terme "matrice extracellulaire" bien connu dans le domaine de l'art est développé dans la partie introductive. Au sens de la présente invention, "substance entraînant une désorganisation de la matrice extracellulaire" désigne toute substance agissant sur l'intégrité de la matrice, notamment excerçant une action dégradative ou déstabilisante totale ou partielle sur l'un au moins des constituants de ladite matrice ou sur les liens qui unissent ces divers constituants. Dans le cadre de la présente invention, on peut avoir recours à une substance connue mais également, s'il s'agit d'une substance de nature protéique, à un mutant comportant une ou plusieurs mutations par addition, délétion et/ou substitution d'un ou plusieurs acides aminés dc la protéine native, un fragment fonctionnel ou encore une protéine chimère provenant de la fusion de séquences d'origines diverses. Aux fins de la présente invention, ladite substance peut également être modifiée par voie chimique, enzymatique ... etc, dans le but d'augmenter son activité, sa stabilité ou encore son tropisme à l'égard d'un type cellulaire particulier. Le choix d'une substance en usage dans la présente invention est large. A titre indicatif, elle peut être choisie parmi les substances ayant une activité collagénase, dispase, trypsine, pronase.

Selon un mode de réalisation avantageux, on préfère avoir recours à une substance capable d'hydrolyser les polysaccharides habituellement présents dans les matrices extracellulaires, et tout particulièrement l'acide hyaluronique. A cet égard, une substance ayant une activité hyaluronidase convient tout particulièrement à la mise en oeuvre de l'invention. Les hyaluronidases sont décrites dans Kreil (Protein Sci., 1995, *4*: 1666-1669). Il peut s'agir d'une hyaluronidase dérivant d'une hyaluronate glycanohydrolase de mammifère, de reptile et d'hyménoptère, d'une hyaluronate glycanohydrolase de glande salivaire de sangsue ou d'une hyaluronate lyase bactérienne notamment de streptocoque, pneumocoque et clostridium. Parmi celles-ci, on préfère tout particulièrement la hyaluronidase de testicules bovins. On aura de préférence recours à une substance présentant un degré d'homologie avec la séquence d'une hyaluronidase ou un fragment fonctionnel de celle-ci d'au moins 70 %, de manière avantageuse d'au moins 90 % et, de manière préférée d'au moins 95 %, l'important étant que l'activité hyaluronidase soit conservée. Cette activité enzymatique peut être évaluée par les techniques conventionnelles telles que celles décrites dans Hynes et Ferretti (Methods Enzymol., 1994, *235:* 606-616) ou Bailey et Levine (J. Pharm. Biomed. Anal., 1993, *11*: 285-292).

La substance composant le produit de combinaison selon l'invention peut être une substance disponible commercialement, de préférence acceptable d'un point de vue pharmaceutique. Selon une autre approche, il est possible de la produire par voie recombinante par les techniques classiques dans ce domaine de l'art. Enfin, on peut aussi envisager d'introduire la séquence codante pour ladite substance dans l'acide nucléique d'intérêt ou un vecteur d'expression sous le contrôle des éléments appropriés à son expression dans une cellule ou un organisme hôte. Ce dernier peut alors être administré préalablement ou simultanément à l'acide nucléique d'intérêt. La mise en oeuvre de ce mode de réalisation spécifique est à la portée de l'homme de l'art.

Dans le cadre de la présente invention, l'acide nucléique d'intérêt peut être un oligonucléotide, un acide ribonucléique ou un acide désoxyribonucléique, sens ou antisens. Ces dénominations sont classiquement utilisées en biologie moléculaire. En bref, "sens" désigne un acide nucléique ayant une séquence homologue ou identique à une séquence cible alors que antisens fait référence à un acide nucléique ayant une séquence homologue ou identique à une séquence complémentaire à une séquence cible. Conformément aux buts poursuivis par la présente invention, l'acide nucléique d'intérêt comporte au moins un gène d'intérêt et des éléments permettant son expression dans une cellule ou un organisme hôte. L'acide nucléique d'intérêt est sous la forme d'une particule virale infectieuse ou d'un vecteur synthétique, sélectionné parmi les vecteurs à lipides cationiques ou à polymères cationiques, étant entendu que ladite substance n'est pas la collagénase.

Selon une variante par ailleurs préférée, l'acide nucléique d'intérêt est porté par un vecteur adénoviral défectif pour la réplication (incapable de réplication autonome dans une cellule hôte). La technologie des adénovirus est décrite dans l'état de la technique (voir par exemple Graham et Prevec in Methods in Molecular Biology, 1991, vol 7, p 109-128, ed E.J. Murey, The Human Press Inc). Avantageusement, le vecteur adénoviral utilisé dans le cadre de la présente invention, dérive du génome d'un adénovirus, comprend au moins les ITRs (Inverted Terminal Repeat) et une séquence d'encapsidation et est dépourvu de tout ou partie de la région adénovirale E1. Il peut en outre être dépourvu de tout ou partie de la région adénovirale E3. Cependant, selon un mode avantageux, on préfère conserver la partie de la région E3 codant pour des polypeptides permettant l'échappement au système immunitaire de l'hôte, notamment la glycoprotéine gp19k (Gooding et al., Critical Review of Immunology, 1990, *10* : 53-71). En outre, il peut comprendre des délétions ou mutations supplémentaires, touchant notamment tout ou partie d'une ou plusieurs régions sélectionnées parmi les régions E2, E4, L1, L2, L3, L4 et L5 (voir par exemple la demande internationale WO 94/28152). Pour illustrer ce point, on peut citer la mutation thermosensible affectant le gène DBP (pour DNA Binding Protein en anglais) de la région E2A (Ensinger et al., J. Virol., 1972, *10* : 328-339). Une autre variante ou une combinaison intéressante consiste à déléter la région E4 à l'exception des séquences codant pour les cadres de lecture ouverts (ORF) 6 et 7 (ces délétions limitées ne nécessitent pas de complémentation de la fonction E4 ; Ketner et al., Nucleic Acids Res., 1989, *17* : 3037-3048). Préférentiellement, le ou les gènes d'intérêt sont insérés dans le vecteur en remplacement des régions adénovirales délétées, notamment de E1. Dans le cas où l'on met en oeuvre plusieurs gènes d'intérêt, ils peuvent être insérés au même endroit ou à des endroits différents du génome viral, être sous le contrôle des mêmes éléments de régulation ou d'éléments indépendants et, le cas échéant, ils peuvent être en orientation réverse les uns par rapport aux autres afin de minimiser les phénomènes d'interférence au niveau de leur expression. Le génome du vecteur adénoviral recombinant peut être préparé par les techniques de biologie moléculaire ou par recombinaison homologue (voir WO 96/17070).

La propagation des vecteurs adénoviraux utilisés dans le cadre de la présente invention, est effectuée dans une lignée de complémentation capable de fournir *en trans* la ou les fonctions défectueuses afin de produire les polypeptides nécessaires à la constitution des particules virales infectieuses. Par exemple, on aura recours à la lignée 293 pour complémenter la fonction E1 (Graham et al., J. Gen. Virol., 1977, *36*: 59-72) ou les lignées décrites dans la demande internationale WO 97/04119 pour une double complémentation. On peut également employer une lignée cellulaire appropriée et un virus auxilliaire pour complémenter l'ensemble des fonctions défectives. Les particules virales produites sont récupérées de la culture cellulaire et, éventuellement purifiées par les techniques de l'art (gradient de chlorure de césium, étapes chromatographiques ....).

Le vecteur adénoviral utilisé dans le cadre de la présente invention, peut dériver du génome d'un adénovirus d'origine humaine, canine, aviaire, bovine, murine, ovine, porcine ou simienne ou encore d'un hybride comprenant des fragments de génome adénoviral de différentes origines. On peut citer plus particulièrement les adénovirus CAV-1 ou CAV-2 d'origine canine, DAV d'origine aviaire ou encore Bad de type 3 d'origine bovine (Zakharchuk et al., Arch. Virol., 1993, *128*: 171-176 ; Spibey et Cavanagh, J. Gen. Virol., 1989, *70*: 165-172 ; Jouvenne et al., Gene, 1987, *60*: 21-28 ; Mittal et al., J. Gen. Virol., 1995, *76*: 93-102). Cependant, on préférera un vecteur adénoviral d'origine humaine dérivant de préférence d'un adénovirus de sérotype C, notamment de type 2 ou 5.

En ce qui concerne l'acide nucléique d'intérêt, il peut coder pour un ARN antisens et/ou un ARNm qui sera ensuite traduit en un polypeptide d'intérêt thérapeutique. Il peut être de type génomique, ADN complémentaire (ADNc) ou mixte (minigène, dans lequel au moins un intron est délété), être homologue ou hétérologue à la cellule hôte. Le polypeptide pour lequel il code peut correspondre à tout ou partie d'une protéine telle que trouvée dans la nature (protéine native ou tronquée) ou un mutant présentant des propriétés biologiques améliorées et/ou modifiées. II peut également s'agir d'un polypeptide chimère provenant de la fusion de séquences d'origines diverses. L'acide nucléique d'intérêt peut être obtenu par synthèse chimique ou par clonage (criblage de banque d'ADN à l'aide de sondes appropriées, PCR...) et peut être modifié par les techniques conventionnelles de biologie moléculaire.

Dans le cadre de la présente invention, il peut être avantageux d'utiliser un gène d'intérêt codant pour une cytokine ou une lymphokine (interféron α, β ou γ, interleukine (IL), notamment l'IL-2, l'IL-6, l'IL-10 ou encore l'IL-12, un facteur nécrosant des tumeurs (TNF), un facteur stimulateur de colonies (GM-CSF, C-CSF, M-CSF...), un récepteur cellulaire ou nucléaire (notamment ceux reconnus par le virus HIV), un ligand de récepteur, une protéine impliquée dans une maladie génétique (facteur VII, facteur VIII, facteur IX, dystrophine, insuline, protéine CFTR (Cystic Fibrosis Transmembrane Conductance Regulator), hormone de croissance....), une enzyme (uréase, rénine, thrombine....), un inhibiteur d'enzyme (α1-antitrypsine, antithrombine III, inhibiteurs de protéases virales...), un polypeptide à effet anti-tumoral capable d'inhiber au moins partiellement l'initiation ou la progression de tumeurs ou cancers (ARN anti-sens, anticorps, inhibiteur agissant au niveau de la division cellulaire ou des signaux de transduction, produit d'expression d'un gène suppresseur de tumeur, par exemple p53 ou Rb, protéine stimulant le système immunitaire, antigène associé à une tumeur, notamment MUC-1 et E6, E7, L1, L2 d'un virus à papillome HPV....), un antigène du complexe majeur d'histocompatibilité des classes I ou II ou un polypeptide agissant sur l'expression des gènes correspondants, un polypeptide capable d'inhiber une infection virale, bactérienne ou parasitaire ou son développement (polypeptide immunoprotecteur, épitope antigénique, anticorps, variant trans-dominant...), un produit cytotoxique (thymidine kinase de virus simplex de l'herpès 1 (TK-HSV-1), ricine, toxine cholérique, diphtérique.....), une immunotoxine ou encore un polypeptide marqueur (β-galactosidase, luciférase....). Il est à signaler que cette liste n'est pas limitative et que d'autres gênes peuvent également être employés.

Par ailleurs, l'acide nucléique d'intérêt en usage dans la présente invention peut également comprendre un gène de sélection permettant de sélectionner ou identifier les cellules transfectées. On peut citer les gènes *néo* (codant pour la néomycine phosphotransférase) conférant une résistance à l'antibiotique G418, *dhfr* (Dihydrofolate Réductase), CAT (Chloramphenicol Acetyl transférase), *pac* (Puromycine Acétyl-Transferase) ou encore *gpt* (Xanthine Guanine Phosphoribosyl Transferase). D'une manière générale, les gènes de sélection sont connus de l'homme de l'art.

Le ou les gènes portés par l'acide nucléique d'intérêt sont placés sous le contrôle des éléments nécessaires à leur expression dans la cellule ou l'organisme hôte. Il s'agit des éléments permettant leur transcription en ARN et la traduction d'un ARNm en polypeptide. Parmi ceux-ci, le promoteur revêt une importance particulière. Il peut être isolé d'un gène quelconque d'origine eucaryote ou même virale et peut être constitutif ou régulable. Alternativement, il peut s'agir du promoteur naturel du gène en question. Par ailleurs, il peut être modifié de manière à améliorer l'activité promotrice, supprimer une région inhibitrice de la transcription, rendre un promoteur constitutif régulable ou vice versa, introduire un site de restriction... On peut mentionner, à titre d'exemples, les promoteurs viraux CMV (Cytomégalovirus), RSV (Rous Sarcoma Virus), du gène TK du virus HSV-1, précoce du virus SV40 (Simian Virus 40), adénoviral MLP (Major Late Promoter) ou encore les promoteurs eucaryotes des gènes PGK (Phospho Glycerate kinase) murin ou humain, α1-antitrypsine (foie-spécifique), immunoglobuline (lymphocyte-spécifique), surfactant, CFTR (poumon spécifique) ou actine (muscle spécifique). Bien entendu, l'acide nucléique d'intérêt peut en outre comprendre des éléments additionnels améliorant l'expression (séquence intronique, séquence signal, séquence de localisation nucléaire, séquence terminatrice de la transcription, site d'initiation de la traduction de type IRES ou autre....) ou encore à sa maintenance dans la cellule hôte (origine de réplication ...). De tels éléments sont connus de l'homme de l'art.

Comme indiqué précédemment, l'acide nucléique d'intérêt et la substance entraînant une désorganisation de la matrice extracellulaire compris dans le produit de combinaison selon l'invention peuvent être utilisés simultanément, consécutivement ou de manière étalée dans le temps. Simultanément fait référence à une co-administration. Dans ce cas, ces deux composantes essentielles peuvent être mélangées préalablement à l'administration ou peuvent être administrées en même temps dans la cellule ou l'organisme hôte. Il est également possible de les administrer consécutivement c'est à dire l'une après l'autre, quelle que soit la composante du produit de combinaison selon l'invention administrée en premier. Enfin, on peut avoir recours à un mode d'administration étalé dans le temps ou intermittent qui s'arrête et reprend à intervalle régulier ou non. On indique que les voies et les sites d'administration des deux composantes peuvent être différents. Selon un mode de réalisation particulièrement préféré, la substance entraînant une désorganisation de la matrice extracellulaire est administrée avant l'acide nucléique, la voie d'administration des deux composantes étant de préférence semblable (par exemple intramusculaire dans les deux cas). L'intervalle de temps entre les injections n'est pas critique et peut être défini par l'homme de l'art. On peut recommander un intervalle de 10 min à 72 h, avantageusement de 30 min à 48 h, de préférence de 1 à 24 h et, de manière tout à fait préférée, de 1 à 6 h.

Par ailleurs, le produit de combinaison selon l'invention peut également être associé à une ou plusieurs molécule(s) destinée à améliorer l'administration d'acide nucléique. Il peut s'agir de molécules ayant un effet protecteur sur l'acide nucléique (protection à l'égard de la dégradation cellulaire), améliorant sa pénétration ou son expression dans la cellule hôte (peptide fusogène, signal de localisation nucléaire...), permettant le ciblage d'un type cellulaire particulier (ligand ou anticorps reconnaissant une protéine de surface cellulaire...), ou prolongeant l'effet thérapeutique (agent immunosuppresseur....). Il peut être également associé à des agents facilitant la transfection (protéines ...).

Le produit de combinaison selon l'invention peut être préparé en vue d'une administration locale parentérale ou par voie digestive. On peut citer notamment la voie intragastrique, sous-cutanée, intracardiaque, intraveineuse, intrapéritonéale, intrasynoviale, intratumorale, intrapulmonaire, intranasale ou intratrachéale et, tout particulièrement, intramusculaire. L'administration peut avoir lieu par toute technique de l'art (injection, voie orale, aérosol, instillation...), en dose unique ou répétée une ou plusieurs fois après un certain délai d'intervalle. La voie d'administration peut être adaptée selon le gène d'intérêt à transférer et la maladie à traiter. La formulation peut inclure des véhicules (excipients, adjuvants...) pharmaceutiquement acceptables. De préférence, la substance entraînant une désorganisation de la matrice extracellulaire et l'acide nucléique d'intérêt sont en solution dans un tampon approprié à un usage pharmaceutique qui peut être hyper, hypo ou isotonique. Les tampons envisageables sont variés. On peut citer à titre illustratif, une solution saline physiologique (NaCl 0,9 %), une solution saline non physiologique (NaCl 1,8 %), une solution Hépes-Ringer, de Lactate-Ringer, un tampon à base de Tris-HCl (Tris-HCl 10 mM pH 7,5 à 8, EDTA 1 mM ; Tris-HCl 10 mM pH 7,5 à 8, MgCl₂ 1 mM), un tampon phosphate (tampon phosphate H₂O Krebs), une solution de sucre (glucose, saccharose, tréhalose....) ou simplement de l'eau.

Avantageusement, le produit de combinaison selon l'invention contient une quantité de substance entraînant une désorganisation de la matrice extracellulaire suffisante pour améliorer la diffusion de l'acide nucléique d'intérêt, en particulier au niveau ou à proximité du site d'injection. La quantité nécessaire varie en fonction de différents paramètres, par exemple de la substance choisie, de la voie d'administration, du tissu cible, de l'individu à traiter, ou de l'étendue de la zone à traiter. S'agissant de la hyaluronidase, la quantité adéquate peut correspondre à celles habituellement utilisées dans les applications d'agent de diffusions de molécules pharmacologiques. A titre indicatif, la dose à mettre en oeuvre est comprise entre 1 et 10⁴ unités internationales (IU), avantageusement entre 1 et 10³ et de préférence entre 10 et 500 IU.

Par ailleurs la quantité d'acide nucléique d'intérêt peut être définie en fonction du gêne thérapeutique et du vecteur utilisé. S'agissant de la variante selon laquelle on met en oeuvre des particules adénovirales, celles-ci sont de préférence formulées sous forme de doses comprises entre 10⁴ et 10¹⁴ ufp (unités formant des plages), avantageusement 10⁵ et 10¹³ ufp et, de préférence, 10⁶ et 10¹² ufp. Dans le cas où l'acide nucléique d'intérêt est sous forme d'un ADN plasmidique, les quantités peuvent varier de 0,05 à 100 mg et avantageusement de 0,1 à 10 mg.

La présente invention concerne également l'utilisation à des fins thérapeutiques ou vaccinales d'un produit de combinaison selon l'invention pour la préparation d'un médicament destiné au traitement du corps humain ou animal par thérapie génique ainsi que l'utilisation d'une substance entraînant une désorganisation de la matrice extracellulaire pour améliorer la diffusion, le transfert et/ou l'expression d'un acide nucléique d'intérêt dans une cellule ou un organisme hôte. L'organisme hôte est avantageusement un mammifère, de préférence l'homme. Quant à la cellule hôte, il peut s'agir d'une cellule primaire ou tumorale d'une origine hématopoïétique (cellule souche totipotente, leucocyte, monocyte, lymphocyte, macrophage....), musculaire (cellule satellite, myocyte, myoblaste....), hépatique, épithéliale, fibroblaste, pulmonaire ou trachéale. Selon une première possibilité, le médicament ou la substance peut être administré directement *in vivo* (par exemple par injection intraveineuse, intramusculaire, dans une tumeur accessible, dans les voies respiratoires par aérosol...). On peut également adopter l'approche *ex vivo* qui consiste à prélever des cellules du patient (cellules souches de la moëlle osseuse, lymphocytes du sang périphérique...), à les traiter *in vitro* avant de les réadminister au patient. La substance entraînant une désorganisation de la matrice extracellulaire est de préférence une hyaluronidase et, selon un mode préféré déjà cité, elle est administrée préalablement à l'acide nucléique d'intérêt.

L'invention s'étend également à l'utilisation d'un produit de combinaison, selon l'invention pour la fabrication d'un médicament destiné à prévenir et/ou à traiter des maladies. Les maladies ciblées sont notamment les maladies génétiques (hémophilie, mucoviscidose, diabète, myopathie de Duchenne ou de Becker...), les cancers et tumeurs (éventuellement induits par des oncogènes ou des virus), les infections virales (hépatites B et C, SIDA, herpès....). Un produit de combinaison administrable par voie intramusculaire ou intraveineuse et associant la hyaluronidase et un acide nucléique exprimant le gène de la dystrophine convient tout particulièrement au traitement par thérapie génique de la dystrophie musculaire de Duchenne. En effet, les muscles atteints sont le siège d'une invasion de tissu conjonctif (et donc, de matrice extracellulaire). Un traitement par la hyaluronidase pourrait permettre d'accroître les possibilités de diffusion de l'acide nucléique d'intérêt et sa pénétration dans les fibres musculaires protégées par une matrice extracellulaire compacte et dense. Une autre application préférée est le traitement de la mucoviscidose, pour lequel la hyaluronidase pourrait permettre à la fois la réduction du mucus et la diffusion de l'acide nucléique thérapeutique. Dans ce cas, on peut envisager l'administration dans les voies respiratoires (aérosol, instillation....) ou intraveineuse d'un acide nucléique exprimant la protéine CFTR.

La présente invention est illustrée, sans pour autant être limitée, par les exemples qui suivent:

### Exemple 1 : Effet de la hyaluronidase sur l'administration intramusculaire d'un adénovirus codant pour le gène de 1a luciférase

L'acide nucléique d'intérêt comportant le gène rapporteur luciférase placé sous le contrôle du promoteur MLP d'Ad2 et des séquences de polyadénylation du virus SV40, est porté par un vecteur adénoviral délété des régions E1 et E3. La construction finale désignée pTG8509 est réalisée selon les techniques générales de génie génétique et clonage moléculaire détaillées dans Maniatis et al. (1989, *supra).* La première étape consiste à cloner la cassette d'expression du gène luciférase dans un plasmide bactérien. On peut par exemple introduire le gène luciférase dans le plasmide pTG6580 (décrit dans la demande internationale WO 94/28152), qui comprend dans une structure p poly II (Lathe et al., 1987, *supra)* l'ITR 5' et la région d'encapsidation de l'Ad5 (nt 1 à 458 tels que divulgués dans la banque de données Genebank sous la référence M73260), le promoteur MLP de l'Ad2, un polylinker, le signal de terminaison de la transcription polyA de SV40 (nt 2543 à 2618 tels que divulgués dans la banque de données Genebank sous la référence J02400) suivis des séquences Ad5 des nt 4047 à 6241. Puis, la cassette luciférase est insérée dans le squelette adénoviral à la place de la région E1 par recombinaison homologue avec le vecteur pTG3602 (voir demande internationale WO 96/17070) linéarisé par *Cla*I et dans la souche *E*. *coli* BJ5183 (Hanahan, J. Mol. Biol., 1983, *166*: 557-580).

Les particules adénovirales correspondantes sont produites après transfection de la lignée 293 (ATCC CRL1573) par le fragment *Pac*I isolé du vecteur pTG8509 et portant le génome adénoviral et récupérées de la culture après lyse cellulaire (habituellement 3 cycles consécutifs de congélation-décongélation). Les particules adénovirales AdTG8509 sont amplifiées et purifiées sur gradient de chlorure de césium avant leur administration *in vivo.*

Le produit de combinaison testé associe 10⁹ unités infectieuses d'AdTG8509 et des doses variables de hyaluronidase de testicules bovins obtenues par dilution d'une préparation lyophilisée (type VI-S, Sigma à 3100 UI) dans NaCl 0,9 %.

Dix souris femelles balb/c âgées de 7 semaines sont réparties au hasard en 5 groupes d'expérimentation recevant dans les deux muscles *tibialis anterior* 25 ml d'une solution de hyaluronidase dont les doses s'échelonnent de 0 UI, 0,05 UI, 0,1 UI, 1 UI et 10 UI et 3 heures après, 25 ml d'une suspension virale d'AdTG8509 titrant 10⁹ unités infectieuses. Le transfert du gène luciférase est évalué après réssection de chaque muscle traité, une semaine après les injections de la hyaluronidase et du vecteur. Les animaux sont sacrifiés par dislocation cervicale et les muscles sont immédiatement prélevés et congelés dans l'azote liquide et stockés à -80°C. L'activité luciférase est mesurée au luminomètre (Microlumat LB 96P, Berthold) après broyage du muscle entier et extraction de l'enzyme (Kit Promega). Cette activité enzymatique se traduit en émission de photons exprimée en unités de lumière par minute (RLU). L'administration de hyaluronidase à des doses supérieures à 1 UI s'accompagne d'une augmentation significative de l'expression du gène luciférase par rapport au groupe témoin (0 UI). Le facteur d'amplification est d'environ 2 chez les animaux traités par 1 UI de hyaluronidase bovine préalablement à l'injection des adénovirus recombinants et 4 chez ceux ayant reçu 10 UI de l'enzyme.

L'expérience est répétée dans des souris femelles C57 BL/10 agées de 7 semaines en mettant en oeuvre des doses supérieures de hyaluronidase. On constitue 5 groupes de 3 animaux traités par des doses variables de hyaluronidase bovine pour un volume fixe d'injection de 25 ml puis 3 heures après par 10⁹ unités infectieuses d'adénovirus AdTG8509 (25 ml). Les cinq groupes d'animaux recoivent respectivement 0, 1, 10, 25 et 50 UI de hyaluronidase diluée dans NaCl 0,9 %. Comme précédemment, enzyme et virus sont injectés consécutivement dans les deux muscles *tibialis anterior* des souris. Toutes les souris C57 BL/10 traitées par la hyaluronidase expriment des niveaux supérieurs de luciférase par rapport au groupe témoin (0 UI). Un facteur d'amplification d'environ 10 est obtenu pour le produit associant les adénovirus recombinants et 10 UI de hyaluronidase.

### Exemple 2 : Effet de la hyaluronidase sur l'administration intrachéale d'un adénovirus codant pour le gène luciférase

Les souris C57 BL/10 sont injectées par voie intratrachéale avec différentes doses de hyaluronidase (0, 0.1, 1, 10 et 50 UI dans 25 µl de tampon PBS par injection) trois heures avant l'administration par voie intratrachéale de 10⁸ UI de AdTG8509, distribuées dans 25µl de tampon PBS par injection.

L'activité luciférase est déterminée conformément aux dispositions de l'exemple 1, dans la trachée et dans les poumons, deux jours après l'administration du vecteur adénoviral.

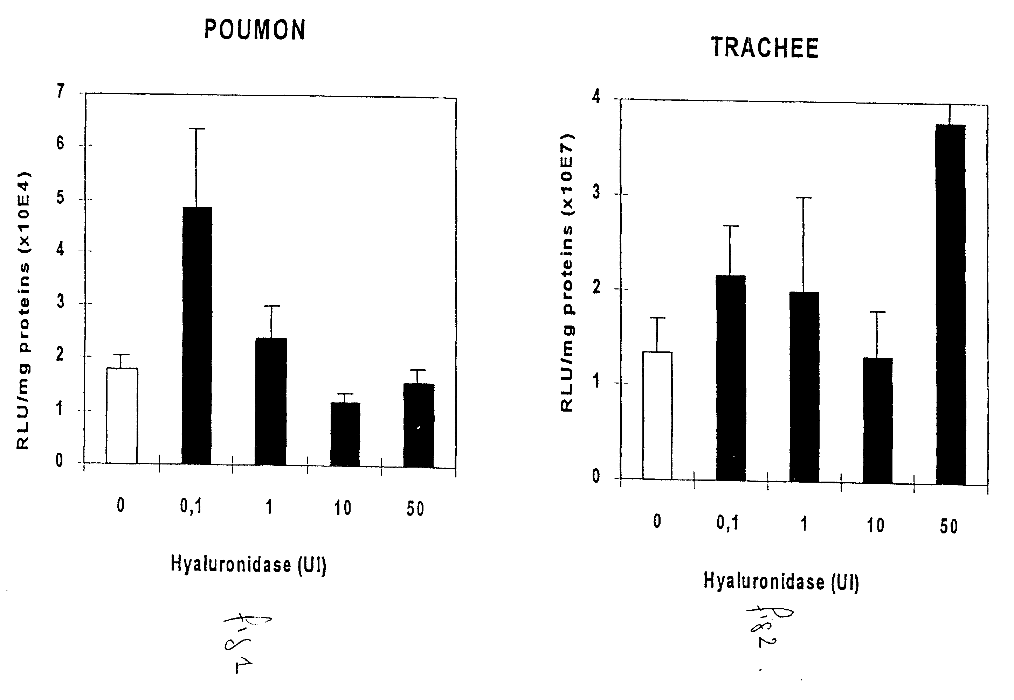

Les résultats sont présentés sur la figure 1. Ceux-ci indiquent que l'activité luciférase est clairement augmentée en présence du hyaluronidase, tant dans les poumons qu'au niveau de la trachée. Un effet lié à la dose de hyaluronidase est clairement observé dans le cas de l'expression au niveau de la trachée.

Les résultats observés semblent indiquer que l'expression du gène luciférase est plus faible dans les poumons que dans la trachée. Ce résultat inhabituel pourrait s'expliquer par une réaction de bronchoconstriction ou d'obstruction au niveau des poumons liées à la première administration de hyaluronidase dans un animal de petite taille.

Ces résultats indiquent toutefois que le prétraitement par la hyaluronidase permet d'améliorer l'efficacité de l'expression des gènes portés par l'adénovirus administré de manière subséquente, au niveau des voies respiratoires. L'utilisation du mode d'administration par aérosolisation devrait permettre de limiter l'effet négatif des administrations répétées par voie intratrachéale chez la souris en raison du volume plus réduit des compositions administrées.

## Revendications

1. Produit de combinaison comprenant au moins une substance entraînant une désorganisation de la matrice extracellulaire d'une cellule et au moins un acide nucléique d'intérêt, pour une administration simultanée, consécutive ou étalée dans le temps, ledit acide nucléique étant sous la forme d'une particule virale infectieuse ou d'un vecteur synthétique, sélectionné parmi les vecteurs à lipides cationiques ou à polymères cationiques, étant entendu que ladite substance n'est pas la collagénase.

2. Produit de combinaison selon la revendication 1, **caractérisé en ce que** ladite substance entraînant une désorganisation de la matrice extracellulaire d'une cellule est une substance capable d'hydrolyser l'acide hyaluronique de ladite matrice.

3. Produit de combinaison selon la revendication 2, **caractérisé en ce que** ladite substance capable d'hydrolyser l'acide hyaluronique dérive d'une hyaluronidase.

4. Produit de combinaison selon la revendication 3, **caractérisé en ce que** ladite hyaluronidase dérive d'une hyaluronate glycanohydrolase de mammifère, de reptile ou d'hyménoptère, d'une hyaluronate glycanohydrolase de glande salivaire de sangsue ou d'une hyaluronate lyase bactérienne notamment de streptocoque, pneumocoque ou clostridium et, en particulier, de la hyaluronidase de testicules bovins.

5. Produit de combinaison selon l'une des revendications 1-4, **caractérisé en ce que** l'acide nucléique d'intérêt est sous forme d'un vecteur adénoviral recombinant défectif pour la réplication.

6. Produit de combinaison selon la revendication 5, **caractérisé en ce que** le vecteur adénoviral dérive du génome d'un adénovirus, comprend au moins les ITRs et une séquence d'encapsidation et est dépourvu de tout ou partie de la région adénovirale E1.

7. Produit de combinaison selon la revendication 6, **caractérisé en ce que** le vecteur adénoviral est en outre dépourvu de tout ou partie de la région adénovirale E3.

8. Produit de combinaison selon la revendication 6 ou 7, **caractérisé en ce que** le vecteur adénoviral est en outre dépourvu de tout ou partie d'une ou plusieurs régions sélectionnées parmi les régions E2, E4, L1, L2, L3, L4 et L5.

9. Produit de combinaison selon l'une des revendications 5 à 8, **caractérisé en ce que** le vecteur adénoviral dérive du génome d'un adénovirus d'origine humaine, canine, aviaire, bovine, murine, ovine, porcine ou simienne ou encore d'un hybride comprenant des fragments de génome adénoviral de différentes origines.

10. Produit de combinaison selon l'une des revendications 5 à 9, **caractérisé en ce que** le vecteur adénoviral dérive du génome d'un adénovirus humain de type 5.

11. Produit de combinaison selon l'une des revendications 1 à 10, **caractérisé en ce que** l'acide nucléique d'intérêt code pour un ARN antisens ou un polypeptide d'intérêt thérapeutique.

12. Produit de combinaison selon la revendication 11, **caractérisé en ce que** le polypeptide d'intérêt thérapeutique est séléctionné parmi une cytokine, un récepteur cellulaire ou nucléaire, un ligand, un facteur de coagulation, la protéine CFTR, l'insuline, la dystrophine, une hormone de croissance, une enzyme, un inhibiteur d'enzyme, un polypeptide à effet anti-tumoral, un polypeptide capable d'inhiber une infection bactérienne, parasitaire ou virale et, notamment le VIH, un anticorps, une toxine, une immunotoxine et un marqueur.

13. Produit de combinaison selon l'une des revendications 1 à 12, **caractérisé en ce qu'**il est formulé dans un véhicule acceptable d'un point de vue pharmaceutique.

14. Produit de combinaison selon l'une des revendications 1 à 13, **caractérisé en ce qu'**il comprend une quantité de ladite substance entraînant une désorganisation de la matrice extracellulaire comprise entre 1 et 10⁴ unités internationales (IU), avantageusement entre 1 et 10³ et de préférence entre 10 et 500 IU.

15. Produit de combinaison selon l'une des revendications 1 à 14, **caractérisé en ce en ce que** ladite substance entraînant une désorganisation de la matrice extracellulaire est administrée avant l'acide nucléique d'intérêt.

16. Utilisation d'un produit de combinaison selon l'une des revendications 1 à 15, pour la préparation d'un médicament destiné au traitement du corps humain ou animal par thérapie génique.

17. Utilisation selon la revendication 16, **caractérisée en ce que** ledit produit de combinaison comprend une substance entraînant une désorganisation de la matrice extracellulaire dérivant d'une hyaluronidase, notamment de la hyaluronidase de testicules bovins, et un acide nucléique d'intérêt sous forme d'un vecteur adénoviral véhiculé par une particule adénovirale infectieuse.

18. Utilisation selon la revendication 16 ou 17, **caractérisée en ce que** ladite substance entraînant une désorganisation de la matrice extracellulaire est administrée avant l'acide nucléique.

19. Utilisation selon l'une des revendications 16 à 18, pour la préparation d'un médicament destiné au traitement de la myopathie de Duchenne, par administration intramusculaire ou intraveineuse d'une hyaluronidase et d'un acide nucléique d'intérêt exprimant le gène codant pour la dystrophine.

20. Utilisation selon l'une des revendications 16 à 18, pour la préparation d'un médicament destiné au traitement de la mucoviscidose, par administration intraveineuse ou dans les voies respiratoires d'une hyaluronidase et d'un acide nucléique exprimant le gène codant pour la protéine CFTR.

21. Utilisation d'une substance entraînant une désorganisation de la matrice extracellulaire pour améliorer la diffusion, le transfert et/ou l'expression d'un acide nucléique d'intérêt dans une cellule ou un organisme hôte, ledit acide nucléique étant véhiculé par une particule virale infectieuse ou sous la forme d'un vecteur synthétique sélectionné parmi les vecteurs à lipides cationiques ou à polymères cationiques, étant entendu que ladite substance n'est pas la collagénase.

## Claims

1. Combination product which comprises at least one substance which leads to disorganization of the extracellular matrix of a cell, and at least one nucleic acid of interest, for simultaneous, separate or sequential administration, with the said nucleic acid being in the form of an infectious viral particle or a synthetic vector, selected from cationic lipid vectors or cationic polymer vectors, provided that said substance is not collagenase.

2. Combination product according to Claim 1, **characterized in that** the said substance which leads to disorganization of the extracellular matrix of a cell is a substance which is able to hydrolyse the hyaluronic acid of the said matrix.

3. Combination product according to Claim 2, **characterized in that** the said substance which is able to hydrolyse hyaluronic acid is derived from a hyaluronidase.

4. Combination product according to Claim 3, **characterized in that** the said hyaluronidase is derived from a mammalian, reptilian or hymenopteran hyaluronate glycanohydrolase, from a hyaluronate glycanohydrolase from the salivary gland of the leech, or from a bacterial, in particular streptococcal, pneumococcal or clostridial, hyaluronate lyase, in particular from bovine testicular hyaluronidase.

5. Combination product according to one of claims 1-4, **characterized in that** the nucleic acid of interest is in the form of a recombinant adenoviral vector which is defective for replication.

6. Combination product according to Claim 5, **characterized in that** the adenoviral vector is derived from the genome of an adenovirus, comprises at least the ITRs and an encapsidation sequence and lacks all or part of the E1 adenoviral region.

7. Combination product according to Claim 6, **characterized in that** the adenoviral vector additionally lacks all or part of the E3 adenoviral region.

8. Combination product according to Claim 6 or 7, **characterized in that** the adenoviral vector additionally lacks all or part of one or more regions selected from the E2, E4, L1, L2, L3, L4 and L5 regions.

9. Combination product according to one of Claims 5 to 8, **characterized in that** the adenoviral vector is derived from the genome of an adenovirus of human, canine, avian, bovine, murine, ovine, porcine or simian origin or else from a hybrid which comprises adenoviral genome fragments of different origins.

10. Combination product according to one of Claims 5 to 9, **characterized in that** the adenoviral vector is derived from the genome of a human type 5 adenovirus.

11. Combination product according to one of Claims 1 to 10, **characterized in that** the nucleic acid of interest encodes an antisense RNA or a polypeptide of therapeutic interest.

12. Combination product according to Claim 11, **characterized in that** the polypeptide of therapeutic interest is selected from a cytokine, a cell or nuclear receptor, a ligand, a coagulation factor, the CFTR protein, insulin, dystrophin, a growth hormone, an enzyme, an enzyme inhibitor, a polypeptide wich has an antineoplastic effect, a polypeptide which is capable of inhibiting a bacterial, parasitic or viral, in particular HIV, infection, an antibody, a toxin, an immunotoxin and a marker.

13. Combination product according to one of Claims 1 to 12, **characterized in that** it is formulated in an excipient which is acceptable from the pharmaceutical point of view.

14. Combination product according to one of Claims 1 to 13, **characterized in that** it comprises a quantity of the said substance which leads to disorganization of the extracellular matrix which is between 1 and 10⁴ international units (IU), advantageously between 1 and 10³ IU and preferably between 10 and 500 IU.

15. Combination product according to one of Claims 1 to 14, **characterized in that** the said substance which leads to disorganization of the extracellular matrix is administered before the nucleic acid of interest.

16. Use of a combination product according to one of Claims 1 to 15 for preparing a medicament which is intended for treating the human or animal body by means of gene therapy.

17. Use according to Claim 16, **characterized in that** the said combination product comprises a substance which leads to disorganization of the extracellular matrix and which is derived from a hyaluronidase, in particular bovine testicular hyaluronidase, and a nucleic acid of interest in the form of an adenoviral vector which is transported by an infectious adenoviral particle.

18. Use according to Claim 16 or 17, **characterized in that** the said substance which leads to disorganization of the extracellular matrix is administered before the nucleic acid.

19. Use according to one of Claims 16 to 18, for preparing a medicament which is intended for treating Duchenne's muscular dystrophy by the intramuscular or intravenous administration of a hyaluronidase and a nucleic acid of interest which expresses the gene encoding dystrophin.

20. Use according to one of Claims 16 to 18, for preparing a medicament which is intended for treating cystic fibrosis by the intravenous administration, or administration into the airways, of a hyaluronidase and a nucleic acid which expresses the gene encoding the CFTR protein.

21. Use of a substance which leads to disorganization of the extracellular matrix for improving the diffusion, the transfer and/or the expression of a nucleic acid of interest into or in a cell or a host organism, with the said nucleic acid being transported by an infectious viral particle or in the form of a synthetic vector selected from cationic lipid vectors or cationic polymer vectors, it being understood that the said substance is not collagenase.

## Patentansprüche

1. Kombinationsprodukt, umfassend mindestens eine Substanz, die eine Desorganisation der extrazellulären Matrix einer Zelle nach sich zieht, und mindestens eine interessierende Nukleinsäure für eine gleichzeitige, aufeinanderfolgende oder zeitlich gestaffelte Verabreichung, wobei die Nukleinsäure in Form eines infektiösen viralen Partikels oder eines synthetischen Vektors vorliegt, der ausgewählt ist unter den Vektoren mit kationischen Lipiden oder mit kationischen Polymeren, mit der Maßgabe, dass die Substanz nicht Kollagenase ist.

2. Kombinationsprodukt nach Anspruch 1, **dadurch gekennzeichnet, dass** die Substanz, die eine Desorganisation der extrazellulären Matrix einer Zelle nach sich zieht, eine Substanz ist, welche die Hyaluronsäure der Matrix hydrolysieren kann.

3. Kombinationsprodukt nach Anspruch 2, **dadurch gekennzeichnet, dass** die Substanz, die die Hyaluronsäure hydrolysieren kann, von einer Hyaluronidase abstammt.

4. Kombinationsprodukt nach Anspruch 3, **dadurch gekennzeichnet, dass** die Hyaluronidase von einer Hyaluronatglycanohydrolase von Säugern, Reptilien oder Hautflüglern, von einer Hyaluronatglycanohydrolase der Speicheldrüse von Blutegeln oder von einer bakteriellen Hyaluronatlyase, insbesondere von Streptokokken, Pneumokokken oder Clostridium, und insbesondere von der Hyaluronidase von Rindertestikeln abstammt.

5. Kombinationsprodukt nach einem der Ansprüche 1 - 4, **dadurch gekennzeichnet, dass** die interessierende Nukleinsäure in Form eines replikationsdefizienten rekombinanten adenoviralen Vektors vorliegt.

6. Kombinationsprodukt nach Anspruch 5, **dadurch gekennzeichnet, dass** der adenovirale Vektor vom Genom eines Adenovirus abstammt, mindestens die ITRs und eine Enkapsidationssequenz umfasst und ihm die ganze oder ein Teil der adenoviralen Region E1 fehlt.

7. Kombinationsprodukt nach Anspruch 6, **dadurch gekennzeichnet, dass** dem adenoviralen Vektor darüber hinaus die ganze oder ein Teil der adenoviralen Region E3 fehlt.

8. Kombinationsprodukt nach Anspruch 6 oder 7, **dadurch gekennzeichnet, dass** dem adenoviralen Vektor darüber hinaus die ganze oder ein Teil einer oder mehrerer Regionen fehlt, die ausgewählt sind aus den Regionen E2, E4, L1, L2, L3, L4 und L5.

9. Kombinationsprodukt nach einem der Ansprüche 5 bis 8, **dadurch gekennzeichnet, dass** der adenovirale Vektor von einem Genom eines Adenovirus menschlichen, Hunde-, Vogel-, Rinder-, Mäuse-, Schafs-, Schweine- oder Affen-Ursprungs oder auch von einem Hybrid abstammt, das Fragmente eines adenoviralen Genoms aus verschiedenen Ursprüngen umfasst.

10. Kombinationsprodukt nach einem der Ansprüche 5 bis 9, **dadurch gekennzeichnet, dass** der adenovirale Vektor vom Genom eines Typ 5-Human-Adenovirus abstammt.

11. Kombinationsprodukt nach einem der Ansprüche 1 bis 10, **dadurch gekennzeichnet, dass** die interessierende Nukleinsäure für eine Antisinn-RNA oder ein Polypeptid von therapeutischem Interesse kodiert.

12. Kombinationsprodukt nach Anspruch 11, **dadurch gekennzeichnet, dass** das Polypeptid von therapeutischem Interesse ausgewählt ist aus einem Cytokin, einem zellulären oder nukleären Rezeptor, einem Liganden, einem Koagulationsfaktor, dem Protein CFTR, Insulin, Dystrophin, einem Wachstumshormon, einem Enzym, einem Enzyminhibitor, einem Polypeptid mit Antitumor-Wirkung, einem Polypeptid, das eine bakterielle, parasitäre oder virale Infektion und insbesondere das HIV hemmen kann, einem Antikörper, einem Toxin, einem Immunotoxin und einem Marker.

13. Kombinationsprodukt nach einem der Ansprüche 1 bis 12, **dadurch gekennzeichnet, dass** es in einem Vehikel formuliert ist, das unter pharmazeutischem Gesichtspunkt annehmbar ist.

14. Kombinationsprodukt nach einem der Ansprüche 1 bis 13, **dadurch gekennzeichnet, dass** es eine Menge der Substanz, die eine Desorganisation der extrazellulären Matrix nach sich zieht, von 1 bis 10⁴ internationalen Einheiten (IE), vorteilhaft 1 bis 10³ und bevorzugt 10 bis 500 IE umfasst.

15. Kombinationsprodukt nach einem der Ansprüche 1 bis 14, **dadurch gekennzeichnet, dass** die Substanz, die eine Desorganisation der extrazellulären Matrix nach sich zieht, vor der interessierenden Nukleinsäure verabreicht wird.

16. Verwendung eines Kombinationsproduktes nach einem der Ansprüche 1 bis 15 für die Herstellung eines Medikaments, das zur Behandlung des menschlichen oder tierischen Körpers durch Gentherapie bestimmt ist.

17. Verwendung nach Anspruch 16, **dadurch gekennzeichnet, dass** das Kombinationsprodukt eine Substanz, die eine Desorganisation der extrazellulären Matrix nach sich zieht und von einer Hyaluronidase, insbesondere der Hyaluronidase von Rindertestikeln abstammt, und eine interessierende Nukleinsäure in Form eines adenoviralen Vektors, der von einem infektiösen adenoviralen Partikel getragen wird, umfasst.

18. Verwendung nach Anspruch 16 oder 17, **dadurch gekennzeichnet, dass** die Substanz, die eine Desorganisation der extrazellulären Matrix nach sich zieht, vor der Nukleinsäure verabreicht wird.

19. Verwendung nach einem der Ansprüche 16 bis 18 für die Herstellung eines Medikaments, das zur Behandlung der Duchenne-Myopathie durch intramuskuläre oder intravenöse Verabreichung einer Hyaluronidase und einer interessierenden Nukleinsäure bestimmt ist, die das Gen exprimiert, das für Dystrophin kodiert.

20. Verwendung nach einem der Ansprüche 16 bis 18 für die Herstellung eines Medikaments, das zur Behandlung von Mukoviszidose durch intravenöse Verabreichung oder Verabreichung in den Atemwegen einer Hyaluronidase und einer Nukleinsäure bestimmt ist, welche das Gen exprimiert, das für das Protein CFTR kodiert.

21. Verwendung einer Substanz, die eine Desorganisation der extrazellulären Matrix nach sich zieht, um die Diffusion, den Transfer in eine Zelle oder einen Wirtsorganismus und/oder die Expression darin einer interessierenden Nukleinsäure zu verbessern, wobei die Nukleinsäure von einem infektiösen viralen Partikel oder in Form eines synthetischen Vektors, der ausgewählt ist aus Vektoren mit kationischen Lipiden oder mit kationischen Polymeren, getragen wird, mit der Maßgabe, dass die Substanz nicht Kollagenase ist.
